# EUROPÄISCHE PATENTSCHRIFT

(11) **EP 0 983 220 B1**
(45) Veröffentlichungstag und Bekanntmachung des Hinweises auf die Patenterteilung: **24.07.2002**
(21) Anmeldenummer: 98929301.4
(22) Anmeldetag: 12.05.1998
(51) Int. Cl.: C07C 29/19, C07C 31/27

(54) **VERFAHREN ZUR HERSTELLUNG VON CYCLOALIPHATISCHE GRUPPEN ENTHALTENDEN ALKOHOLEN**
METHOD FOR PRODUCING ALCOHOLS CONTAINING CYCLOALIPHATIC GROUPS
PROCEDE DE PRODUCTION D'ALCOOLS CONTENANT DES GROUPES CYCLOALIPHATIQUES

(30) Priorität: 16.05.1997 DE 19720606
(43) Veröffentlichungstag der Anmeldung: 08.03.2000
(73) Patentinhaber: BASF AKTIENGESELLSCHAFT, 67056 Ludwigshafen (DE)
(72) Erfinder: FISCHER, Rolf, D-69121 Heidelberg (DE); PINKOS, Rolf, D-67098 Bad Dürkheim (DE); WULFF-DÖRING, Joachim, D-67227 Frankenthal (DE)
(74) Vertreter: Isenbruck, Günter, Dr.
(86) Internationale Anmeldenummer: EP9802778
(87) Internationale Veröffentlichungsnummer: WO9852892

(56) Entgegenhaltungen:
- DATABASE WPI Section Ch, Week 7707 Derwent Publications Ltd., London, GB; Class E15, AN 77-11893Y XP002075812 & JP 52 000 242 A (TOYO SPINNING CO.) in der Anmeldung erwähnt

## Beschreibung

Die Erfindung betrifft ein Verfahren zur Herstellung von cycloaliphatische Gruppen enthaltenden Alkoholen durch Hydrierung von aromatische Kerne enthaltenden Carbonsäuren oder Anhydriden oder Estern davon in Gegenwart eines Katalysators.

Verfahren zur Hydrierung von aromatischen Carbonsäuren zu cycloaliphatischen Alkoholen sind bekannt. Dabei wird zunächst der aromatische Kern zur cycloaliphatischen Gruppe hydriert und anschließend jede Carboxylgruppe in eine Hydroxymethylengruppe überführt.

In der JP-A-77/000242 (1977) ist ein Verfahren zur Herstellung von 1,4-Cyclohexandimethanol beschrieben. Dabei wird zunächst Terephthalsäure an einem Katalysator aus 5 % Rhodium auf Kohle kernhydriert. Anschließend werden die Carboxylgruppen durch Hydrierung an einem Kupfer/Chrom-Katalysator in die Alkoholgruppen überführt. Die Umsetzung kann auch in einem kontinuierlich betriebenen Reaktor durchgeführt werden, der die beiden Katalysatoren räumlich voneinander getrennt hintereinander enthält.

In der JP-A-06/228028 (1994) ist ein Verfahren zur Herstellung von 1,4-Cyclohexandimethanol beschrieben, bei dem zunächst der aromatische Kern von Terephthalsäure an einem Katalysator aus 5 % Ru auf Al₂O₃ hydriert wird, und die erhaltene Verbindung anschließend an einem Trägerkatalysator, der Ru und/oder Rh und Sn enthält, zu 1,4-Cyclohexandimethanol hydriert wird.

In der US 5,395,986 ist ein Verfahren beschrieben, bei dem die bereits kernhydrierte cycloaliphatische Dicarbonsäure an einem Kupfer/Chrom-Katalysator zu 1,4-Cyclohexandimethanol hydriert wird.

Zweistufige Verfahren sind aufwendig in der Verfahrensführung und unter anderem durch den Einsatz zweier unterschiedlicher Katalysatoren in zwei Reaktionsstufen kostspielig.

Aufgabe der vorliegenden Erfindung ist daher die Bereitstellung eines Verfahrens zur katalytischen Hydrierung von aromatische Kerne enthaltenden Carbonsäuren zu cycloaliphatische Gruppen enthaltenden Alkoholen, das die Nachteile der bekannten Verfahren vermeidet.

Die Aufgabe wird erfindungsgemäß gelöst durch Bereitstellung eines Verfahrens zur Herstellung von cycloaliphatische Gruppen enthaltenden Alkoholen durch Hydrierung von aromatische Kerne enthaltenden Carbonsäuren oder Anhydriden oder Estern davon in Gegenwart eines Katalysators, wobei die Hydrierung einstufig ausgeführt wird und der Katalysator mindestens ein Element aus der Gruppe bestehend aus Pd, Ru und Pt und das Element Re in jeweils metallischer oder oxidischer Form enthält.

Es wurde erfindungsgemäß gefunden, daß die Hydrierung einstufig ausgeführt werden kann, wenn sie an speziellen, mindestens bimetallischen oder bimetalloxidischen Katalysatoren durchgeführt wird.

Der Katalysator kann dabei als Voll- oder Trägerkatalysator eingesetzt werden. Für den Einsatz als Trägerkatalysator eignen sich alle bekannten Trägermaterialien, beispielsweise Aktivkohlen, SiO₂, Al₂O₃, TiO₂, ZrO₂, Tonerden wie Montmorillonite, Zeolithe oder Gemische davon. Vorzugsweise wird der Katalysator als Vollkatalysator eingesetzt.

Beispielsweise kann ein Katalysator eingesetzt werden, der herstellbar ist durch Reduktion einer wäßrigen Aufschlämmung und/oder Lösung von Oxiden, Oxidhydraten, Carbonaten, Nitraten, Carboxylaten, Chelaten, Sulfaten, Phosphaten und/oder Halogeniden der eingesetzten Elemente.

Der Katalysator enthält ein Element aus der Gruppe bestehend aus Pd, Ru oder Pt, bevorzugt Pt.

Als Element der 7. Gruppe des Periodensystems der Elemente wird Re verwendet.

Der Katalysator enthält zusätzlich mindestens ein weiteres Element aus einer der Gruppen 3 und 5 bis 12 und 14 und den Lanthaniden des Periodensystems der Elemente in metallischer oder oxidischer Form.

Bevorzugte Beispiele für das mindestens eine weitere Element sind Sn, V, Cr, Mo, W, Mn, Fe, Ru, Os, Co, Ni, Pd, Cu, Ag, Au, Zn, La und Ce. Die Elemente können als Oxide, Oxidhydrate, Carbonate, Nitrate, Carboxylate, Chelate von 1,3-Diketoverbindungen, Sulfate, Phosphate oder Halogenide eingesetzt werden.
Die Elemente Re und Pd, Ru und Pt werden vorzugsweise als Oxide, Oxidhydrate, Carbonate, Nitrate, Sulfate, Halogenide, Boride, Carboxylate wie Acetate, Chelate von 1,3-Diketoverbindungen wie Enolate von Acetylacetonat oder Benzylacetonat eingesetzt. Besonders bevorzugt sind Oxide und Oxidhydrate wie RuO₂, PdO₂, PtO₂ und Re₂O₇.

Besonders bevorzugt enthält der Katalysator ein Element aus der Gruppe bestehend aus Pd, Ru und Pt, und das Element Re und mindestens ein Element aus der Gruppe 11 des Periodensystems der Elemente in metallischer oder oxidischer Form. Insbesondere enthält der Katalysator Pd, Ru oder Pt neben Re und Ag, Mo oder Au in metallischer oder oxidischer Form. Der Katalysator kann die vorstehend angegebenen Elemente enthalten oder aus ihnen bestehen. Vorzugsweise besteht er aus diesen Elementen in jeweils metallischer oder oxidischer Form.

Die erfindungsgemäß eingesetzten Katalysatoren können hergestellt werden, indem die entsprechenden Verbindungen der Elemente zusammen vorgelegt werden und bevorzugt mit Wasserstoff reduziert werden. Die Reduktion kann jedoch auch nacheinander durchgeführt werden. Beispielsweise werden in Wasser PtO₂, eine Re-Verbindung und gegebenenfalls eine Verbindung mindestens eines weiteren Elements vorgelegt und mit Wasserstoff reduziert. Die Reduktion erfolgt dabei vorzugsweise bei Temperaturen von 100 bis 700°C, besonders bevorzugt 200 bis 600°C, insbesondere 250 bis 500°C. Die so erhaltenen Katalysatoren können nach der Reduktion direkt zur Hydrierung eingesetzt werden. Trägerkatalysatoren können beispielsweise so hergestellt werden, daß sich eine Pt-Komponente, bevorzugt Pt-Oxid oder Pt-Oxidhydrat bereits auf dem Träger befindet, wobei das Pt/Trägergemisch durch Tränkung oder gemeinsame Fällung von Pt-Oxidvorläufer oder Pt-Oxidhydratvorläufer und Trägermaterial und anschließender Calcinierung hergestellt werden kann. Die Re-Verbindung sowie das mindestens eine weitere Element können durch Tränkung oder Fällung zusätzlich aufgebracht werden. Dabei kann das Pt-Oxid oder Pt-Oxidhydrat auf dem Träger vorher reduziert worden sein. Nach den vorstehenden Herstellungsbedingungen liegen die Katalysatoren als Gemische der Elemente oder teilweise oder überwiegend als intermetallische Verbindungen vor.

Vorzugsweise beträgt das Gewichtsverhältnis von Pt zu Re beziehungsweise von Pt zu dem gegebenenfalls vorhandenen weiteren Element 100 bis 0,01, besonders bevorzugt 50 bis 0,05, insbesondere 10 bis 0,1.

Als Carbonsäuren können bei der Hydrierung beliebige aromatische Kerne enthaltende Carbonsäuren, deren Anhydride oder Ester eingesetzt werden. Die Carbonsäure kann dabei eine beliebige Anzahl an Carboxylgruppen aufweisen. In der Regel enthält sie 1 bis 3 Carboxylgruppen. Insbesondere handelt es sich um eine Dicarbonsäure. Die Carboxylgruppen können durch einen Alkylenrest vom aromatischen Kern getrennt sein. In diesem Fall enthält der Alkylenrest vorzugsweise 1 bis 5 Kohlenstoffatome. Besonders bevorzugt handelt es sich um eine aromatische Carbonsäure, bei der die Carboxylgruppen direkt an den aromatischen Kern gebunden sind. Als aromatische Kerne kommen alle geeigneten aromatischen Kerne, wie Benzolkeme, Naphthalinkerne und höherkondensierte aromatische Kerne in Betracht. Vorzugsweise ist der aromatische Kern ein Benzolkern. Der aromatische Kern kann durch Hydroxylgruppen, C₁₋₁₂-Alkylreste, C₁₋₁₂-Alkoxyreste oder C₁₋₁₂-Hydroxyalkylreste substituiert sein. Liegen im Molekül oder in den Substituenten Carbonylfunktionen vor, so werden diese bei der Hydrierung ebenfalls reduziert. Liegen in den Substituenten Kohlenstoffdoppel- oder Kohlenstoffdreifachbindungen vor, so werden auch diese zu den entsprechenden gesättigten Verbindungen reduziert.

Beispiele für geeignete Carbonsäuren sind Monocarbonsäuren wie Benzoesäure oder Dicarbonsäuren wie Phthalsäure oder Terephthalsäure. Als Ester können beispielsweise die Methyl-, Ethyl-, Propyl- oder Butylester eingesetzt werden. Die Carbonsäuren können auch als Anhydride, beispielsweise Phthalsäureanhydrid eingesetzt werden. Die entstehenden Alkohole können in einer Vielzahl von Anwendungen eingesetzt werden, beispielsweise als Zwischenprodukte oder Alkoholkomponenten bei der Herstellung von Polymeren.

Bevorzugt werden Terephthalsäure bzw. deren Ester eingesetzt.

Die Carbonsäuren oder Anhydride oder Ester davon können unverdünnt oder als Suspension oder Lösung eingesetzt werden. Als Lösungsmittel eignen sich alle unter den Reaktionsbedingungen inerten Stoffe wie Wasser, Dioxan, Tetrahydrofuran, Ethylenglykolether, Kohlenwasserstoffe wie Hexan oder Alkohole wie Methanol, Ethanol, Propanol, Butanol oder das Reaktionsprodukt selbst. Bevorzugt werden Wasser und die bei der Reaktion entstehenden Alkohole als Lösungsmittel eingesetzt.

Die Hydrierung erfolgt einstufig, d.h. aromatische Kerne und Carboxylgruppen werden gleichzeitig am Katalysator hydriert.

Dabei wird die Hydrierung vorzugsweise bei Temperaturen von 30 bis 300°C, besonders bevorzugt 80 bis 215°C, insbesondere 100 bis 190°C durchgeführt. Der Druck beträgt vorzugsweise 1 bis 350 bar. Bei Umsetzungen in der Gasphase beträgt der Druck vorzugsweise 1 bis 80 bar, bei Umsetzungen in der Flüssigphase vorzugsweise 20 bis 330 bar, besonders bevorzugt 100 bis 300 bar.

Die Hydrierung kann kontinuierlich oder diskontinuierlich durchgeführt werden. Je nach Reaktionsführung wird der Katalysator dabei in einer geeigneten Form eingesetzt. Bei der diskontinuierlichen Verfahrensführung kann der Katalysator beispielsweise in Pulverform als Suspension eingesetzt werden. Bei der kontinuierlichen Verfahrensführung kann der Katalysator als Festbett zur Umsetzung in der Flüssig- oder Gasphase angeordnet sein.

Dabei kann die Hydrierung mit oder ohne Produktrückführung betrieben werden.

Bei der kontinuierlichen Reaktionsführung kann auch eine Folge von Reaktoren eingesetzt werden. Dies kann beispielsweise zur besseren Abfuhr der Reaktionswärme notwendig sein.

Die Erfindung wird durch die nachstehenden Beispiele näher erläutert.

### Beispiele

### BEISPIEL 1

In einem Metallautoklaven wurden 0,1 g PtO₂, 0,2 g Re₂O₇, 0,1 g Silberacetat und 9 g Wasser vorgelegt. Anschließend wurden 60 bar Wasserstoff aufgepreßt, und es wurde unter Rühren auf 270°C aufgeheizt. Nach einer Stunde wurde auf Raumtemperatur abgekühlt, der Autoklav entspannt und 1 g Terephthalsäure zugegeben. Danach wurden 100 bar Wasserstoff aufgepreßt, und es wurde unter Rühren auf 150°C aufgeheizt. Nach 2 Stunden wurde wiederum abgekühlt und entspannt. Der Reaktionsaustrag wurde gaschromatographisch analysiert. Es fanden sich bei vollständigem Umsatz 83 % 1,4-Cyclohexandimethanol. Der Rest bestand überwiegend aus 4-Hydroxymethylcyclohexancarbonsäure und 4-Methylhydroxymethylcyclohexan.

### BEISPIEL 2

Analog Beispiel 1 wurden 0,1 g PtO₂ und 0,2 g Re₂O₇ eingesetzt. Die Hydrierung bei 150°C erfolgte für einen Zeitraum von einer Stunde. Es fanden sich im Austrag 71 % 1,4-Cyclohexandimethanol. Der Rest bestand überwiegend aus 4-Hydroxymethylcyclohexancarbonsäure, 4-Formyl-hydroxymethylcyclohexan und 4-Methylhydroxymethylcyclohexan.

## Patentansprüche

1. Verfahren zur Herstellung von cycloaliphatische Gruppen enthaltenden Alkoholen durch Hydrierung von aromatische Kerne enthaltenden Carbonsäuren oder Anhydriden oder Estern davon in Gegenwart eines Katalysators, **dadurch gekennzeichnet, daß** die Hydrierung einstufig ausgeführt wird und der Katalysator mindestens ein Element aus der Gruppe bestehend aus Pd, Ru und Pt, und das Element Re in jeweils metallischer oder oxidischer Form enthält.

2. Verfahren nach Anspruch 1, **dadurch gekennzeichnet, daß** der Katalysator zusätzlich mindestens ein weiteres Element aus einer der Gruppen 3 und 5 bis 12 und 14 und den Lanthaniden des Periodensystems der Elemente in metallischer oder oxidischer Form enthält.

3. Verfahren nach Anspruch 1 oder 2, **dadurch gekennzeichnet, daß** der Katalysator mindestens ein Element aus der Gruppe 11 des Periodensystems der Elemente in metallischer oder oxidischer Form enthält.

4. Verfahren nach einem der Ansprüche 1 bis 3, **dadurch gekennzeichnet, daß** der Katalysator als weiteres Element Ag, Mo oder Au in jeweils metallischer oder oxidischer Form enthält.

5. Verfahren nach einem der Ansprüche 1 bis 4, **dadurch gekennzeichnet, daß** die Carbonsäure Terephthalsäure ist.

6. Verfahren nach einem der Ansprüche 1 bis 5, **dadurch gekennzeichnet, daß** die Temperatur bei der Hydrierung maximal 200°C beträgt.

7. Verfahren nach einem der Ansprüche 1 bis 6, **dadurch gekennzeichnet, daß** die Hydrierung in Gegenwart von Wasser durchgeführt wird.

8. Verwendung eines Katalysators, wie er in einem der Ansprüche 1 bis 4 definiert ist, zur Hydrierung von aromatische Kerne enthaltenden Carbonsäuren.

## Claims

1. A process for preparing alcohols containing cycloaliphatic groups by hydrogenation of carboxylic acids containing aromatic nuclei, or anhydrides or esters thereof, in the presence of a catalyst, wherein the hydrogenation is carried out in one stage, and the catalyst contains at least one element from the group consisting of Pd, Ru and Pt, and the element Re, in the form of the metal or an oxide in each case.

2. A process as claimed in claim 1, wherein the catalyst additionally contains at least one other element from one of groups 3 and 5 to 12 and 14 and the lanthanides of the Periodic Table of the Elements in the form of the metal or an oxide.

3. A process as claimed in claim 1 or 2, wherein the catalyst contains at least one element from group 11 of the Periodic Table of the Elements in the form of the metal or an oxide.

4. A process as claimed in any of claims 1 to 3, wherein the catalyst contains as other element Ag, Mo or Au, in the form of the metal or an oxide in each case.

5. A process as claimed in any of claims 1 to 4, wherein the carboxylic acid is terephthalic acid.

6. A process as claimed in any of claims 1 to 5, wherein the hydrogenation temperature does not exceed 200°C.

7. A process as claimed in any of claims 1 to 6, wherein the hydrogenation is carried out in the presence of water.

8. The use of a catalyst as defined in any of claims 1 to 4 for the hydrogenation of carboxylic acids containing aromatic nuclei.

## Revendications

1. Procédé de préparation d'alcools qui contiennent des groupes cycloaliphatiques, au moyen d'une hydrogénation d'acides carboxyliques contenant des noyaux aromatiques ou de leurs anhydrides ou de leurs esters, en présence d'un catalyseur, **caractérisé en ce que** l'on réalise l'hydrogénation en une étape et **en ce que** le catalyseur contient au moins un élément choisi dans le groupe formé par Pd, Ru et Pt, ainsi que l'élément Re, chacun étant sous forme métallique ou d'oxyde.

2. Procédé selon la revendication 1, **caractérisé en ce que** le catalyseur contient de plus au moins un élément supplémentaire, sous forme métallique ou d'oxyde, choisi dans un des groupes 3 et 5 à 12 et 14 et les lanthanides de la Classification des Eléments.

3. Procédé selon la revendication 1 ou 2, **caractérisé en ce que** le catalyseur contient au moins un élément, sous forme métallique ou d'oxyde, choisi dans le groupe 11 de la Classification des Eléments.

4. Procédé selon l'une quelconque des revendications 1 à 3, **caractérisé en ce que** le catalyseur contient en tant qu'élément supplémentaire Ag, Mo ou Au, chacun étant sous forme métallique ou d'oxyde.

5. Procédé selon l'une quelconque des revendications 1 à 4, **caractérisé en ce que** l'acide carboxylique est l'acide téréphtalique.

6. Procédé selon l'une quelconque des revendications 1 à 5, **caractérisé en ce que** la température lors de l'hydrogénation est de 200°C au maximum.

7. Procédé selon l'une quelconque des revendications 1 à 6, **caractérisé en ce que** l'on procède à l'hydrogénation en présence d'eau.

8. Mise en oeuvre d'un catalyseur tel qu'il est défini selon l'une quelconque des revendications 1 à 4, pour l'hydrogénation d'acides carboxyliques contenant des noyaux aromatiques.
